# EUROPEAN PATENT SPECIFICATION

(11) **EP 4 463 172 B1**
(45) Date of publication and mention of the grant of the patent: **25.02.2026**
(21) Application number: 23704823.6
(22) Date of filing: 10.01.2023
(51) Int. Cl.: A61K 36/185, A61K 36/45, A61K 36/76, A61K 36/82, A61P 31/14, A61K 9/00, A61K 9/20, A61K 35/56, A61K 35/644

(54) **PRODUCTION OF PROTECTIVE LOZENGE/CHEWABLE TABLET AGAINST SARS-COV-2 VIRUS**
HERSTELLUNG EINER LUTSCHTABLETTE/KAUTABLETTE ZUM SCHUTZ GEGEN SARS-COV-2-VIRUS
PRODUCTION DE PASTILLES / COMPRIMÉS À MÂCHER POUR LA PROTECTION CONTRE LE VIRUS DU SARS-COV-2

(30) Priority: 11.01.2022 TR 202200292
(43) Date of publication of application: 20.11.2024
(73) Proprietor: Tübitak, 06100 Ankara (TR)
(72) Inventor: ALASALVAR, Cesarettin, 41470 Kocaeli (TR); PELVAN PELITLI, Ebru, 41470 Kocaeli (TR); KARAOGLU, Öznur, 41470 Kocaeli (TR); KARADENIZ, Bülent, 41470 Kocaeli (TR); DEMIRTAS, lknur, 41470 Kocaeli (TR); SERHATLI, Müge, 41470 Kocaeli (TR); YESILADA, Erdem, 41470 Kocaeli (TR)
(86) International application number: PCT/IB2023/050183
(87) International publication number: WO 2023/135507

(56) References cited:
- WO-A1-2021/210033
- WO-A1-2021/259772
- US-A1- 2020 138 894
- KALANTARI SARA ET AL: "[beta]-Cyclodextrin-assisted extraction of phenolic compounds from pomegranate (Punica granatum L.) peel: A new strategy for anthocyanin copigmentation", LWT- FOOD SCIENCE AND TECHNOLOGY, ACADEMIC PRESS, UNITED KINGDOM, vol. 151, 12 July 2021 (2021-07-12), XP086765819, ISSN: 0023-6438, [retrieved on 20210712], DOI: 10.1016/J.LWT.2021.112136
- SURUCIC RELJA ET AL: "Computational study of pomegranate peel extract polyphenols as potential inhibitors of SARS-CoV-2 virus internalization", MOLECULAR AND CELLULAR BIOCHEMISTRY, vol. 476, no. 2, 16 November 2020 (2020-11-16), pages 1179 - 1193, XP037362195, ISSN: 0300-8177, DOI: 10.1007/S11010-020-03981-7
- GÜLER HALIL IBRAHIM ET AL: "Targeting CoV-2 spike RBD and ACE-2 interaction with flavonoids of Anatolian propolis by in silico and in vitro studies in terms of possible COVID-19 therapeutics", TURKISH JOURNAL OF BIOLOGY, vol. 45, no. SI-1, 30 August 2021 (2021-08-30), pages 530 - 548, XP055958223, DOI: 10.3906/biy-2104-5

## Description

### Technical Field of the Invention

The present invention relates to producing a mixture of green tea, bilberry, pomegranate peel and black poplar propolis extracts, the effectiveness of which has been proven against SARS-CoV-2 virus by *in vitro* tests, and turning it into lozenges/chewable tablets. It has been proven by *in vitro* tests (on VERO E6 cells) that the formulated mixture is effective against SARS-CoV-2 virus. Cytotoxicity tests were also performed on the developed product. The reason for developing the product in lozenge/chewable tablet form is to provide a protective effect around the mouth and throat, which is the first entry point of the SARS-CoV-2 virus. The combination of herbal extracts with propolis extract were synergically more effective than that of individual extract.

### Prior Art

COVID-19 (Coronavirus disease 2019), which started in China in 2019 and spread all over the world, has become an epidemic that threatens humanity. SARS-CoV-2 (severe acute respiratory syndrome coronavirus-2), which is the cause of COVID-19 disease, is a contagious virus that causes severe respiratory tract infection in humans. SARS-CoV-2 is an enveloped virus infecting humans and some animal species, with single-stranded RNA (ribonucleic acid) as genetic material. This group of viruses causes cold and flu-like symptoms, especially by causing upper respiratory tract infections. Sore throat, dry cough, runny nose, weakness and fatigue are among the most common symptoms [1, 2].

To date, the number of COVID-19 cases worldwide has exceeded 185 million, with more than 4 million deaths [1]. COVID-19 has significantly impacted the health systems of countries at the global level. In addition, it has caused problems in the fields of education and economy, and has greatly affected the food and tourism sectors. Social isolation has also caused psychological problems, by limiting social life of individuals.

The rapid transmission of COVID-19 among humans and its high death rate has led to an urgent need for the development of new treatment methods for the disease.

Although there is no known effective method that can treat COVID-19 all over the world for now, 19 different vaccines have been developed [1]. Clinical trials of possible anti-viral drugs are underway.

Clinical studies have shown that plant extracts are of great importance in the development of drugs for the treatment of viral diseases, thanks to their rich bioactive and phytochemical compounds [3].

Plants synthesize secondary metabolites with a wide range of biological activities in order to develop and survive. Most of these metabolites are chemicals that form a defense mechanism in the plant against biotic stress (insects, parasites, and microbial pathogens) and abiotic stress (low or high temperature, insufficient or excess water, salty environment, heavy metals, and UV light) [4]. For centuries, people have used plant extracts for treatment and protection purposes against diseases. The therapeutic effects of a diverse and numerous plant-derived components against SARS-CoV-2 are being investigated.

Currently, there are supplements that are used to strengthen the immune system. There are products in the form of tablets, capsules, and lozenges containing different combinations of beta-glucan, black elderberry extract, African geranium, turmeric extract, grape seed extract, green tea powder extract, pomegranate peel extract with vitamins and minerals to strengthen the immune system as a food supplement.

Especially in throat infections, lozenge/chewable tablets used to reduce pain and facilitate swallowing cause salivary glands to be activated. In this way, lozenges/chewable tablets that increase the secretion of the natural antimicrobial substance "lysozyme" in mouth can prevent the development and colonization of microorganisms that enter the body through mouth and nose, including the onset of throat infections. On the other hand, it is reported that SARS-CoV-2 type viruses and variants penetrate into cells by attaching to ACE-2 (angiotensin-converting enzyme-2) and TMPRSS2 (transmembrane-protease-serine-2) receptors of cells. It is reported that the cells with the highest concentration of these receptors are found in the oral cavity and nasal mucosa. Therefore, it is reported that lozenge/chewable tablets can play an important role in preventing the attachment and penetration of viruses to these receptors in the oral mucosa.

*The existing patents and applications are summarized below.*
- The patent document numbered AU2020100565 titled *"A composition to boost and enhance immune system and application thereof*" is aimed at increasing the immune system and, unlike the present invention, contains extracts of *Terminalia ferdinandiana, Undaria pinnatifida, Curcuma longa,* and *Vitis vinfera.*
- The patent document numbered US20160095879 titled *"Composition for enhancing immunity",* unlike the present invention, is intended for the development of food supplements, natural preparations or pharmaceutical preparations for regulating general immune system, not for protection against SARS-CoV-2.
- The Japanese patent document numbered JP2005314316A, titled *"Anti-Sars Coronavirus Agent"* against SARS-CoV-2, addresses, unlike the present invention, the development of food products containing proanthocyanidin, catechin, and grape extract, which act as coronavirus agents.
- The Turkish patent document numbered TR2021/01716 titled *"New Options with Antiviral Effectiveness against SARS-CoV-2",* addresses, unlike the present invention, the development of combinations of *Vitis vinifera* seed oil, carvacrol, thymol, and gamma aminobutyric acid, which have a potential anti-viral effect against COVID-19.
- The Turkish patent document numbered TR2020/16536 titled *"Herbal Food Supplement Effective against COVID-19"* addresses the development of fermented liquid extracts with anti-viral activity against COVID-19 obtained from different plants and plant parts, unlike the plants and the bee product in the present invention.
- The patent document numbered US10758587B2 titled *"Formulations for the Treatment of Disorders of the Mouth, Throat, and Respiratory Tract"* contains extracts of *Curcuma longa, Punica granatum,* and *Zingiber officinale.* It is proven by *in vitro* and clinical studies that this mixture is effective in the prevention of upper respiratory tract diseases by preventing the formation of bacterial plaque and in the treatment of upper respiratory tract diseases by increasing the amount of lysozyme in saliva with anti-viral, antioxidant activity and anti-inflammatory effect. The invention subject to application, on the other hand, includes *Punica granatum* extract as well as *Camellia sinensis* extract, *Vaccinium myrtillus* extract, and black poplar propolis extract, which is a bee product (animal source), and its effectiveness against SARS-CoV-2 virus has been proven by *in vitro* experiments.
- The patent document numbered US9173912B2 titled *"Cistus extract containing enriched secondary plant ingredients",* unlike the present invention, defines the extraction recipes of extracts containing a minimum of 40% (w/w) polyphenol obtained from the labdanum *(Cistus creticus L.)* plant. It also reports that the extract containing 60% polyphenol is effective against *Clostridium perfringens, Vibrio cholerae* and influenza A H1N1 viruses. It is stated that the invention can be applied in drop, nasal spray, syrup, tablet, lozenge, and powder forms.
- The patent document numbered US 2012/0244087 A1 titled *"Oral compositions containing a combination of natural extracts and related methods"* is about the preparation of a mixture from the combination of at least three of the plants *Punica granatum, Myristica fragrans, Zingiber officinale,* and *Zizyphus joazeiro* and the use of the mixture in oral care product formulation. It is stated that these oral care products have antimicrobial, anti-inflammatory, and antioxidant properties and are also effective in the treatment of xerostomia with no need for using another antibacterial agent. The peculiarity of the mixture is that it can take the desired form such as liquid, powder, gel, chewable tablet, drop, and film, in order to facilitate application to the oral surface.
- The patent document numbered US20090175971A1 titled *"Methods of using Composition Comprising Pomegranate Extracts against the Common Cold",* unlike the present invention, states that a composition of one or more embodiments of pomegranate may be used to treat the common cold caused by any number of viral agents like adenoviruses, rhinoviruses, coronaviruses, human parainfluenza virus, or any variation of them.
- Patent application WO2021/259772A1 discloses a throat lozenge comprising anthocyanins for use in treating or preventing a SARS-CoV-2 infection.

Most of the prior applications are inventions that reveal anti-viral properties against viruses by strengthening the immune system. Besides that, the previous invention includes, apart from plant and bee product extracts, herbal liquid extracts with potential anti-viral activity against SARS-CoV-2 virus and products consisting of different combinations of synthetic and natural molecules.

Green tea (*Camellia sinensis*) is produced by drying freshly harvested *Camellia sinensis* leaves under hot air or steam without oxidation and fermentation. The most important phenolic compounds in green tea are catechins [epicatechin (EC), epicatechin gallate (ECG), epigallocatechin (EGC), epigallocatechin gallate (EGCG)] [5]. It has been observed that catechins have an important role in the prevention and treatment of cardiovascular diseases, obesity, diabetes, oxidative and inflammatory diseases, bacterial and viral diseases, cancer, and neurological diseases [6,7]. The polyphenols in green tea help reduce body inflammation, have antioxidant and anti-viral properties against SARS-CoV-2. In addition, the phenolic compounds in green tea have anti-viral properties against SARS-CoV-2.

Pomegranate (*Punica granatum L.*)*,* which is grown intensively in the Mediterranean region, is rich in polyphenols such as ellagic tannins (ETs), gallic acid (GA), ellagic acid (EA), punicalin, and anthocyanins [8]. The pomegranate fruit is composed of peel by 40%. Pomegranate peel is very rich in dietary fiber and polyphenols, and also has a very high antioxidant capacity compared to pulp [9]. Studies have shown that pomegranate peel has antioxidant, anti-bacterial [10,11], anti-microbial [12], and anti-viral [13,14] effects as well as anti-inflammatory activity. The ETs, GA, EA, and punicalin found in the pomegranate peel have immune system boosting and anti-viral properties against SARS-CoV-2.

Bilberry (*Vaccinium myrtillus*) is a plant species that grows in the temperate climate zone, whose fruit falls into berry fruits group. Its wild forms grow especially in the Black Sea region, as well as the Marmara Region and Eastern Anatolia flora in Türkiye. Bilberry is very rich in phenolic components, especially anthocyanins (malvidin, delphinidin, and cuomarin) [15,16]. Bilberry has anti-viral properties against SARS-CoV-2, thanks to the phenolic compounds and anthocyanins (malvidin, delphidin, curomarin, etc.) in it.

Propolis (*Propolire*) is a resinous material produced by honey bees to protect their hives and is widely used to strengthen the immune system. Containing phenolic substances such as caffeic acid phenethyl ester (CAPE), caffeic acid, p-coumaric acid, t-cinnamic acid, hydroxycinnamic acid derivatives, and flavonoids (galangin, crisine, quercetin, pinocembrin, pinobanksin), propolis has immune system boosting, anti-inflammatory and anti-viral properties against SARS-CoV-2 [17]. Among the effective components in the phenolic content of black poplar (*Populus nigra*) propolis, CAPE is reported to have a wide spectrum of pharmacological activity in scientific studies conducted. It was observed that CAPE is not found in the other three propolis types (trembling poplar type, 3-methyl quercetin type, and an unknown type) found in Türkiye. In this regard, black poplar propolis extract is extremely important in getting the biological benefits attributed to propolis. It is also reported that pinocembrin, pinobanksin, galangin, and chrysin in black poplar propolis are phenolic components responsible for the anti-microbial effect [18]. Containing phenolic substances such as CAPE, caffeic acid, p-coumaric acid, t-cinnamic acid, hydroxycinnamic acid derivatives) and flavonoids (galangin, chrysin, quercetin, pinocembrin, pinobanksin), black poplar propolis has immune system boosting, anti-inflammatory, and anti-viral properties against SARS-CoV-2.

The present invention relates to the powderization and formulation of extracts in certain proportions, which are rich in phenolic components and are effective against SARS-CoV-2 (anti-viral). Drying and powderization of the bioactive-rich mixture is quite important for preventing deterioration, increasing shelf life, and ease of use.

Prior art informations serve for strengthening the immune system and revealing anti-viral properties against viruses. Besides, the previous invention includes, apart from plant and bee product extracts, herbal liquid extracts with potential anti-viral activity against SARS-CoV-2 virus and products consisting of different combinations of synthetic and natural molecules. Also, unlike the prior art, the present invention includes bee product as well as herbal extracts. The synergistic effect was taken into account.

Black poplar propolis as a bee product adds value to the present invention with its effectiveness against SARS-CoV-2 and other pathogens owing to its CAPE and rich polyphenol content. The effectiveness of black poplar propolis against SARS-CoV-2 is revealed for the first time thanks to this invention. Black poplar propolis, a bee product, was added to the invention in order to get a wide efficacy profile.

The present invention relates to an easy-to-use and low-toxicity food supplement whose anti-viral activity against SARS-CoV-2 is proven by *in vitro* experiments, and which also strengthens the immune system and reduces the amount of virus transmitted through mouth and nose (with 112% neutralizing activity [61% cell viability] at 110 µg/mL concentration]. Also, unlike other inventions, the present invention includes bee product as well as herbal extracts. It was found that the combination of herbal extracts with propolis extract were synergically more effective (~ 7-fold higher) than that of individual extract.

### Technical Problem that Invention Aims to Solve

Although there is no known effective method that can treat COVID-19 across the world for now, 19 different vaccines have been developed so far [1]. Nevertheless, the development of appropriate treatment methods is an urgent need.

This invention relates to a mixture of green tea, bilberry, pomegranate peel, and black poplar propolis extracts, which prevents viral infection targeting host proteins and reduces the activity of SARS-CoV-2 virus transmitted through the upper respiratory tract. The invention was developed in the form of a throat lozenge/chewable tablet in order to provide a protective effect around the mouth and throat, which is the first point of entry of the SARS-CoV-2 virus. The effectiveness of the mixture in the present invention against SARS-CoV-2 virus was tested on VERO E6 cells by *in vitro* methods. In the experiments lasting 160 hours, the mixture neutralizes the virus by 112% (61% cell viability) at 110 µg/mL concentration and by 35% (82% cell viability) at 70 µg/mL concentration.

Herbal substances are metabolized to a great extent under varying pH and enzyme conditions in the digestive system. Therefore, the studies conducted under *in-vitro* conditions are unlikely to reveal systematically the same efficacy throughout the body. However, in case of local application in mouth (as lozenge/chewable tablets) without entering the digestive system, it will be possible to ensure the exact effectiveness of *in-vitro* results. The lozenge/chewable tablet form of the invention allows the active ingredients to have local activity without being metabolized in the digestive system.

Considering that the oral cavity is the main entry point for SARS-CoV-2 and other microorganisms, such a lozenge is expected to play an important role in the prevention of infections.

### Descriptions of the Figures

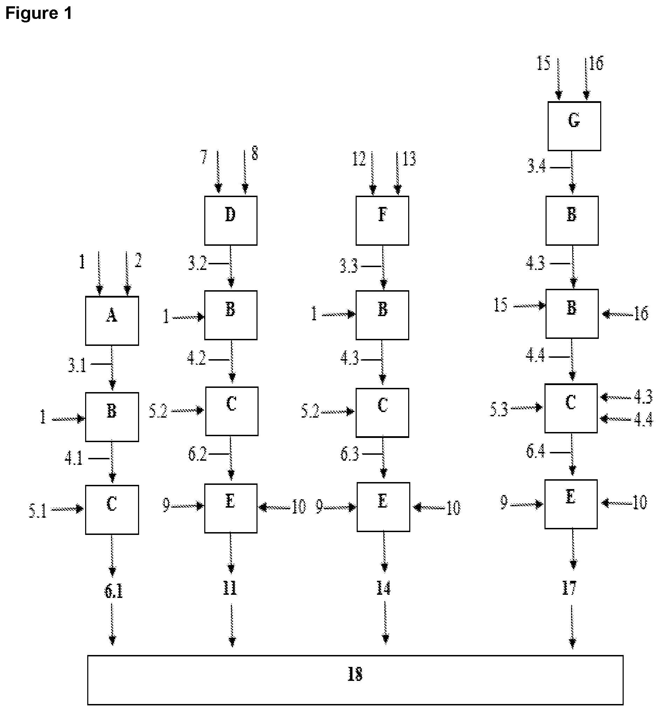

**Figure 1****.** Production of the mixture with anti-viral activity.
**Figure 2****.** Graph of neutralization efficiency of the mixture against SARS-CoV-2 virus on VERO E6 cells, measured by xCELLigence RTCA MP device.

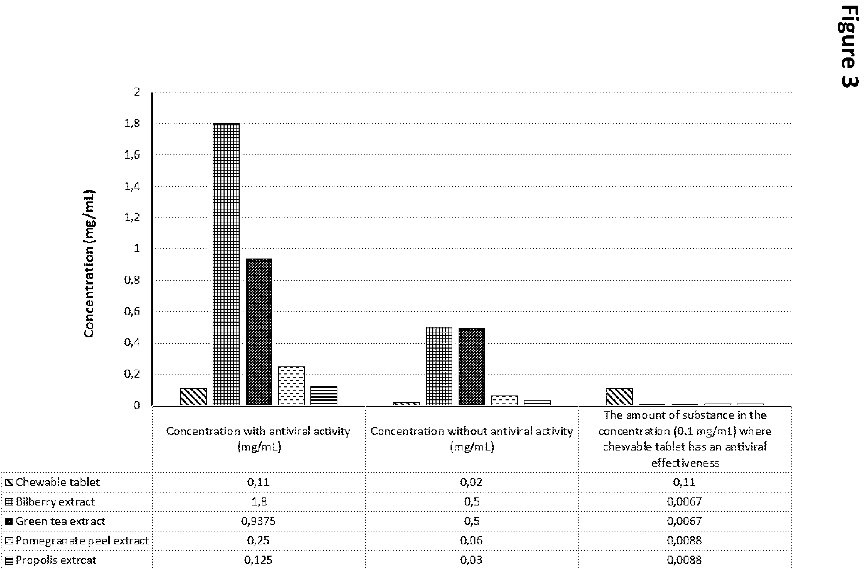

**Figure 3****.** Comparison of the effectiveness of the chewable table and its extracts against the Wuhan variant of SARS-CoV-2 virus using the xCELLigence RTCA-MP system.

### Descriptions of the References in the Figures

**A:** Green Tea Extraction
   1:100% Ethanol
   2: Temperature (A)
   3.1: Filtration
**B:** Filtering Process
   1:100% Ethanol
   4.1: Filtrate
C: Drying Process
   5.1: Temperature (C)
   6.1: Green Tea Extract
**D:** Bilberry Extraction
   7:50% Ethanol
   8: Temperature (D)
   3.2: Filtration
**B:** Filtering Process
   1:100% Ethanol
   4.2: Filtrate
**C:** Drying Process
   5.2: Temperature (C)
   6.2: Bilberry Aqueous Extract
**E:** Freeze Drying Process
   9: Temperature (E)
   10: Maltodextrin
   11: Bilberry Extract
**F:** Pomegranate Peel Extraction
   12: 30% Ethanol
   13: Temperature (F)
   3.3: Filtration
**B:** Filtering Process
   1:100% Ethanol
   4.3: Filtrate
**C:** Drying Process
   5.2: Temperature (C)
   6.3: Pomegranate Peel Aqueous Extract
**E:** Freeze Drying Process
   9: Temperature (E)
   10: Maltodextrin
   14: Pomegranate Peel Extract
**G:** Black Poplar Propolis Extraction
   15: 70% Ethanol
   16: Temperature (G)
   3.4: Filtration
**B:** Filtering Process
   4.3: Filtrate (1)
   4.4: Filtrate (2)
**C:** Drying Process
   5.3: Temperature (C)
   6.4: Aqueous Extract of Black Poplar Propolis
**E:** Freeze Drying Process
   9: Temperature (E)
   10: Maltodextrin
   17: Black Poplar Propolis Extract
   18: Mixture of green tea extract (6.1), bilberry extract (11), pomegranate peel extract (14), and black poplar propolis extract (17)

### Description of the Invention

This invention is defined in the appended claims and relates to a mixture of green tea (6.1), bilberry (11), pomegranate peel (14) and black poplar propolis (17) extracts, which prevents viral infection targeting host proteins and reduces the activity of SARS-CoV-2 virus transmitted through the upper respiratory tract. The invention was developed in the form of a throat lozenge/chewable tablet in order to provide a protective effect around the mouth and throat, which is the first point of entry of the SARS-CoV-2 virus. The effectiveness of the mixture in the present invention against SARS-CoV-2 virus was tested on VERO E6 cells by *in-vitro* methods. In the experiments lasting 160 hours, the mixture neutralizes the virus by 112% (61% cell viability) at 110 µg/mL concentration and by 35% (82% cell viability) at 70 µg/mL concentration.

In the present invention, the development of throat lozenge/chewable tablet is shown in **Example-2.** The present invention contains at least one or a few of the excipients of sorbitol (E420), ascorbic acid (E300), citric acid (E330), tartaric acid (E334), magnesium salts of fatty acids (E470b), microcrystalline cellulose (E460i), maltodextrin, gum arabic (E414), lecithin, bovine gelatine (E441), polyethylene glycol (E1521), glycerin, carboxymethyl cellulose (E466), hydroxypropyl cellulose (E463), lactose, sucrose, sucrolose (E955), saccharose, xylose, glycerol (E422), sodium chloride, natural flavorings (such as mint, lemon flavor), and natural color substances (such as beta-carotene and lycopene).

In the present invention, green tea (6.1), bilberry (11), pomegranate peel (14) and black poplar propolis (17), whose anti-viral activities have been proven by *in-vitro* tests, are subjected to aqueous extraction with water and alcohol solution, and then dried, powderized and formulated in certain proportions.

When used against SARS-CoV-2 virus, the invention has the capacity to neutralize the virus at 110 µg/mL concentration by 112%, with a cell viability of 61%.

Production of Extracts:
✔ *Green Tea Extract (6.1):* Green tea is ground into fine powder and stirred in water bath with a solid:liquid ratio of 1:2 - 1:20 (w:v) (preferably 1:10, w:v), with 100% ethanol (1) at 60 - 95 °C (2) (preferably at 85 °C) for 0.5-2 hours (preferably 1 hour). After the mixture reaches room temperature, it is washed with 100% ethanol (1) while passing through filter (3.1). Then, the ethanol (1) in the filtrate (4.1) is evaporated until it gets dry at 40 °C (5.1) under vacuum. Green Tea Extract (6.1) is produced by this way. In the production of Green Tea Extract (6.1), other techniques such as, but not limited to, supercritical liquid extraction, pressurized liquid extraction, accelerated solvent extraction, and aqueous extraction can be used as an alternative to solid/liquid extraction.
*✔ Bilberry Extract (11):* After grinding the frozen bilberry fruit, it is mixed with 30-70% ethanol (7) (preferably 50%) solvent with a solid:liquid ratio of 1:2 to 1:40 (w:v) (preferably 1:10, w:v) at 20-50 °C (8) (preferably at room temperature) for 0.5 - 2 hours (preferably 1 hour). Then, while the mixture is filtered (3.2), it is washed with 100% ethanol (1), the ethanol (1) in the filtrate (4.2) is evaporated under vacuum at 40 °C (5.2). Bilberry aqueous extract (6.2) is mixed with maltodextrin (10) and lyophilized for 72 hours starting from -40 °C (9) to remove the remaining water. Bilberry extract (11) is produced after this process (E). In the production of (11), other techniques such as, but not limited to, supercritical liquid extraction, pressurized liquid extraction, accelerated solvent extraction, and aqueous extraction can be used as an alternative to liquid extraction.
*✔ Pomegranate Peel Extract (14):* After the dried pomegranate peels are ground into fine powder, they are mixed with 25-75% ethanol (12) (preferably 30%, v/v) solvent in a water bath with a solid:liquid ratio of 1:2 - 1:20 (preferably 1:10, w:v) at 30-70 °C (13) (preferably at 50 °C) for 15 minutes - 2 hours (preferably 30 minutes). After the mixture reaches room temperature, it is passed through filter (3.3), washed with 100% ethanol (1), and the ethanol (1) in the filtrate (4.3) is evaporated under vacuum at 40 °C (5.2). Pomegranate peel aqueous extract (6.3) is mixed with maltodextrin (10) and lyophilized for 72 hours starting from - 40 °C (9) to remove the remaining water. The extract (14) is produced after this process (E). In the production of (14), other techniques such as, but not limited to, supercritical liquid extraction, pressurized liquid extraction, accelerated solvent extraction, and aqueous extraction can be used as an alternative to liquid extraction.
*✔* Black Poplar Propolis *Extract* (17): After raw Black Poplar propolis is ground into fine powder, it is mixed with 50-96% ethanol (15) (preferably 70%, v/v) solvent with a solid:liquid ratio of 1:2 - 1:20 (preferably 1:10, w:v) ) at 20-50 °C (16) (preferably at 24 °C) for 2 hours - 4 hours (preferably 3 hours). The upper part of the mixture is carefully filtered (3.4). The same process is repeated (B). Then, the filtrates (4.3 and 4.4) are combined and the ethanol is evaporated (C) under vacuum at 40 °C (5.3). Black Poplar Aqueous Extract (6.4) is mixed with maltodextrin (10) and lyophilized for 72 hours starting from -40 °C (9) to remove the remaining water. The extract (17) is produced after this process (E). In the production of (17), other techniques such as supercritical liquid extraction, pressurized liquid extraction, accelerated solvent extraction, and aqueous extraction can be used as an alternative to liquid extraction.
*✔* After the extracts 6.1, 11, 14, and 17 are produced separately, they are mixed to ensure the following amount ranges in a chewable lozenge/tablet of the invention: green tea extract (6.1) 20-100 mg, bilberry extract (11) 35-100 mg, pomegranate peel extract (14) 20-50 mg, and black poplar propolis extract (17) 35-75 mg. The resulting mixture is added excipients and put into its final form as a lozenge/chewable tablet. While putting into its final form, the mixture can also be put into other forms such as, but not limited to, soft gel, tablet, and throat spray, among others.
*✔* SARS-CoV-2 virus protective lozenge/chewable tablet contains, by weight of the protective mixture of four extracts, 10-55% green tea extract (6.1), 10-50% bilberry extract (11), 10-55% pomegranate peel extract (15), 10-50% black poplar propolis extract (17), and excipients.
✔ The weight ratios of green tea extract, bilberry extract, pomegranate peel extract and black poplar propolis extract in the lozenge/chewable tablet are 3(±1.5):4(±2):3(±1.5):4(±2), respectively.

**Table-1. Extracts used in SARS-CoV-2 protective mixture and an exemplary formulation**

| **Code** | **Plant** | **Origin** | **Extraction Conditions** | **Amount of active ingredient (at least %)** | **% rate per unit** |
|---|---|---|---|---|---|
| 6.1 | Green Tea | *Camellia sinensis* | Solid:liquid ratio 1:10 (w:v), 85°C, 1 hour, 100% ethanol | EGCG 10% | 10-55 |
| 11 | Bilberry | *Vaccinium myrtillus* | Solid:liquid ratio 1:10 (w:v), room temperature, 1 hour, 50% ethanol | Total Anthocyanin 0.75% | 10-50 |
| 14 | Pomegranate Peel | *Punica granatum* | Solid:liquid ratio 1:10 (w:v), 50 °C, 30 minutes, 30% ethanol | EA* 0.2% | 10-55 |
| 17 | Black Poplar propolis | *Populus nigra propolis* | Solid:liquid ratio 1:10 (w:v), room temperature, 3 | CAPE 0.65% | 10-50 |
| | | | hours, 70% ethanol | | |

| | | | | | |
|---|---|---|---|---|---|
| **The ellagic acid (EA) result is the result of punicalin, valoneic acid dilactone, galagic acid dilactone, and ellagic acid components in ellagic acid equivalents.* **EGCG:** epigallocatechin gallate. **CAPE:** caffeic acid phenethyl ester. | | | | | |

### Example 1:

### Exemplary formulation of the invention:

Chewable tablet formulation effective against SARS-CoV-2

| | |
|---|---|
| Green tea extract, (at least 10% EGCG) | 35 mg |
| Pomegranate peel extract, (at least 0.2% EA*) | 46 mg |
| Bilberry extract, (0.75% anthocyanin) | 35 mg |
| Black Poplar propolis extract, (at least 0.65% CAPE) | 46 mg |
| Sorbitol (E420) | 350 mg |
| Microcrystalline cellulose (E460i) | 29 mg |
| Maltodextrin | 22 mg |
| Flavoring agent (Menthol) | 0.52 mg |
| Magnesium salts of fatty acids (E470b) | 9 mg |
| Polyethylene glycol (E1521) | 4 mg |
| Sucralose | 0.29 mg |

| | |
|---|---|
| **The ellagic acid (EA) result is the result of punicalin, valoneic acid dilactone, galagic acid dilactone, and ellagic acid components in ellagic acid equivalents.* | |

### Example 2

### In-vitro experiments

Anti-viral activity test is conducted based on impedance by the use of xCELLigence MP real-time cell analyzer. In this study, it was made use of VERO E6 cells (ATCC CRL 1586) with ACE₂ receptor to which SARS-CoV-2 virus binds, DMEM as medium and 10% fetal bovine serum (FBS, heat inactivated, Hyclone) + 1% antibiotic-antimycotic solution [19-21] as additives.

VERO E6 cells are placed into a sterile single-use 96-well E-plate cell plate of the xCELLigence RTCA MP device, leaving 25000 cells per well. The device is kept in the incubator throughout the experiment and the seeded cells are placed in the device and grown for 24 hours. The samples prepared in accordance with the sample preparation protocol are incubated with 3.5x10⁵ PFU/mL SARS-CoV-2 and 5% CO₂ for 1 hour at 37 °C. After the incubation, the medium on the cells in the cell growth dish is removed; the samples are added to the wells, and incubated at 37 °C with 5% CO₂ for 160 hours. The data collected at 15-minute intervals are analyzed with RTCA Software Pro software.

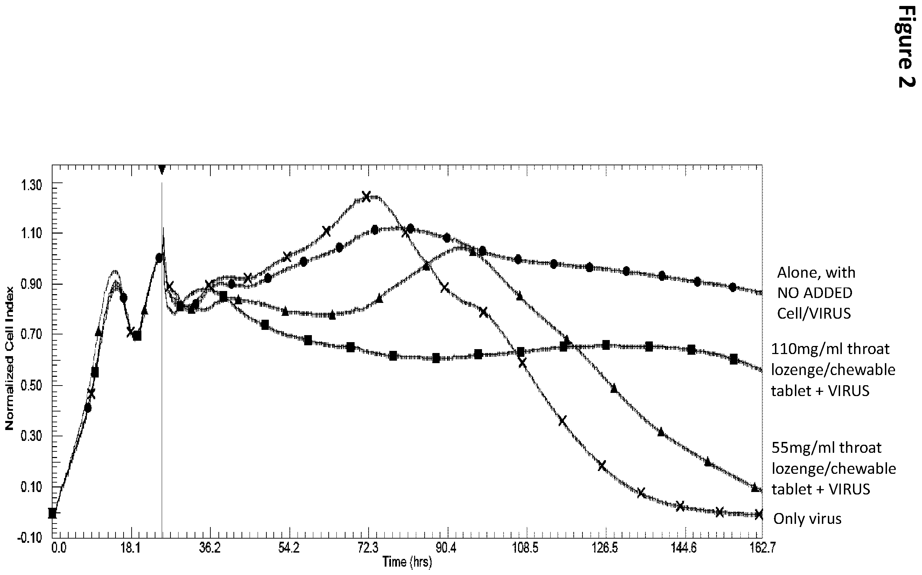

**Figure** 2 shows the graph of neutralization efficiency results of the throat lozenge/chewable tablet sample against SARS-CoV-2 virus on VERO E6 cells, measured by xCELLigence RTCA MP device. The sample and the virus were added to the cell at the 24^{th} hour, and the test results were normalized according to the previous reading range. The experiment continued for 160 hours. In the graph, ( 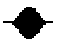 ) represents media control (cell/virus only), ( 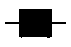 ) throat lozenge/chewable tablet containing 110 µg/mL virus, ( 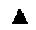 ) throat lozenge/chewable tablet containing 55 µg/mL virus, and ( × ) virus control (3.5x10⁵ PFU)/mL). The throat lozenge/chewable tablet with 110 µg/mL concentration neutralizes the virus by 112% (with 61% cell viability).

### Industrial Application of the Invention

The present invention can be easily produced and commercialized by any company operating in the pharmaceutical and food sector and can be offered for sale as a food supplement in pharmacies or markets.

### References:

[1] World Health Organization, www.who.int. (Accessed 12/07/2021).
[2] Li G., Fan Y., Lai Y., Han T, Li Z., Zhou P., Pan P., Wang W., Hu D., Liu Z., Zhang Q., Wu J. Coronavirus infections and immune responses. J Med Virol (2020) 92(4), 424-432.
[3] Ben-Shabat, S., Yarmolinsky L., Porat D., Dahan A. Antiviral effect of phytochemicals from medicinal plants: Applications and drug delivery strategies. Drug Deliv Transl Res (2020) 10(2), 354-367.
[4] Yang L., Wen, K.S., Ruan, X., Zhao, Y.X., Wei, F., Wang, Q. Response of plant secondary metabolites to environmental factors. Molecules (2018) 23(4), 762.
[5] Pinto M.S. Tea:A new perspective on health benefits. Food Res Int (2013) 53(2), 558-567.
[6] Arab H., Maroofian A., Golestani S., Shafee H., Sohrabi K., Forouzanfar A. Review of the therapeutic effects of Camellia sinensis (green tea) on oral and periodontal health. J. Med. Plant Res (2011) 5(23), 5465-5469.
[7] Chacko S.M., Thambi, P.T., Kuttan, R., Nishigaki I. Beneficial effects of green tea: A literature review. Chin Med (2010) 5(13).
[8] Garcia-Villalba R., Espin J., Aaby K., Alasalvar C., Heinonen M., Jacobs G., Voorspoels S., Koivumaki T., Kroon P., Pelvan E., Saha S., Tomas-Barberan F., Validated method for the characterization and quantification of extractable and nonextractable ellagitannins after acid hydrolysis in pomegranate fruits, juices, and extracts. J Agric Food Chem (2015) 63(29), 6555-6566.
[9] Li Y., Guo C., Jijun Y., Wei J., Xu J., Cheng S., Evaluation of antioxidant properties of pomegranate peel extract in comparison with pomegranate pulp extract. Food Chem (2006) 96(2), 254-260.
[10] Mphahlele R.R., Fawole O.A., Makunga N.P., Opara U.L., Effect of drying on the bioactive compounds, antioxidant, antibacterial and antityrosinase activities of pomegranate peel. BMC complementary and alternative medicine (2016) 16(1), 143.
[11] Malviya S., Jha A., Hettiarachchy N., Antioxidant and antibacterial potential of pomegranate peel extracts. J Food Sci Tech (2014) 51(12), 4132-4137.
[12] Al-Zoreky N.S., Antimicrobial activity of pomegranate (Punica granatum L.) fruit peels. Int J Food Microbiol (2009) 134(3), 244-248.
[13] Tito A., Colantuono A., Pirone L., Pedone E., Intartaglia D., Giamundo G., Conte I., Vitaglione P., Apone F. Pomegranate peel extract as an inhibitor of SARS-CoV-2 spike binding to human ACE2 receptor (in vitro):A promising source of novel antiviral drugs. Front Chem (2021) 9, 638187.
[14] Moradi K.A., Karimi A., Rafieian-Kopaei M.T., Rabiei-Faradonbeh M. , Momtaz H., Pomegranate peel extract inhibits internalization and replication of the influenza virus:An in vitro study. Avicenna J Phytomed (2020) 10(2), 143-151.
[15] Cho M.J., Howard L., Prior R.L., Clark J.R., Flavonol glycosides and antioxidant capacity of various blackberry and blueberry genotypes determined by high-performance liquid chromatography/mass spectrometry. J Sci Food Agr (2005) 85(3), 2149-2158.
[16] Shahidi F., Alasalvar C., Handbook of functional beverages and human health, CRC Press, Boca Raton, 2016.
[17] Berretta A.A., Silveira M.A.D., Capcha J.M.C., Jong D., Propolis and its potential against SARS-CoV-2 infection mechanisms and COVID-19 disease. Biomed (2020) 131, 110622.
[18] Ye 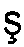 ilada E. Apiterapi: Arilardan Gelen Sa 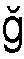 lik. Hayy Yayinlari, istanbul (2015).
[19] Witkowski P., Schuenadel L., Wiethaus, J. Bourquain D., Kurth A., Nitsche, A., Cellular impedance measurement as a new tool for poxvirus titration, antibody neutralization testing and evaluation of antiviral substances. Biochem Biophys Res Commun (2010) 401(1), 37-41.
[20] Charretier C., Saulnier A., Benair L., Armanet C., Bassard I., Daulon S., Bernigaud B., Sousa E., Gonthier C., Zorn E., Vetter E., Saintpierre C., Riou P., Gaillac D. Robust real-time cell analysis method for determining viral infectious titers during development of a viral vaccine production process. J Virol Methods (2018) 252, 57-64.
[21] Teng Z., Kaung X., Wang J., Zhang X. Research on human enterovirus 71 infectivity assay based on a real-time cell analysis. Bing Du Xue Bao (2013) 29(4), 392-7.

## Claims

1. A SARS-CoV-2 virus protective formulation in the form of a lozenge or a chewable tablet **characterized in that** it comprises excipients and a protective mixture of four extracts wherein the amounts by weight of the protective mixture of four extracts are:
- 10-55% by weight green tea extract (6.1) containing at least 10% EGCG as an active ingredient,
- 10-50% by weight bilberry extract (11) containing at least 0.75% total Anthocyanin as an active ingredient,
- 10-55% by weight pomegranate peel extract (14) containing at least 0.2% EA as an active ingredient,
- 10-50% by weight black poplar propolis extract (17) containing at least, 0.65% CAPE as an active ingredient.

2. A lozenge or a chewable tablet according to Claim 1 **characterized in that** the weight ratios of green tea extract, bilberry extract, pomegranate peel extract, and black poplar propolis extract in it are 3(±1.5):4(±2):3(±1.5):4(±2), respectively.

3. A lozenge or a chewable tablet, according to Claim 1 and Claim 2, **characterized in that** the dosage per unit is 20-100 mg for green tea extract, 35-100 mg for bilberry extract, 20-50 mg for pomegranate peel extract, and 35-75 mg for black poplar propolis extract.

4. A lozenge or a chewable tablet according to Claim 1 **characterized in that** it comprises at least one or a few of the excipients of sorbitol (E420), ascorbic acid (E300), citric acid (E330), tartaric acid (E334), magnesium salts of fatty acids (E470b), microcrystalline cellulose (E460i), maltodextrin, gum arabic (E414), lecithin, bovine gelatine (E441), polyethylene glycol (E1521), glycerin, carboxymethyl cellulose (E466), hydroxypropyl cellulose (E463), lactose, sucrose, sucrolose (E955), saccharose, xylose, glycerol (E422), sodium chloride, natural flavorings (such as mint, lemon flavor), and natural color substances (such as beta-carotene and lycopene).

5. A method of production of a lozenge or a chewable tablet according to claim 1, **characterized by** comprising the following steps:
- Green tea extraction (A)
- Bilberry extraction (D)
- Pomegranate peel extraction (F)
- Black poplar propolis extraction (G)
- Mixing the green tea extract (6.1) produced in step (A), the bilberry extract (11) produced in step (D), the pomegranate peel extract (14) produced in step (F), and black poplar propolis extract (17) produced in step (G) in the proportions as stated in Claim 1 (18)
- Adding excipients to the resulting mixture (18)
- Putting the mixture into lozenge or chewable tablet form.

6. A SARS-CoV-2 virus protective formulation in the form of a lozenge or a chewable tablet of any of claims 1 to 4 for use in the prevention and/or in the treatment of a SARS-CoV-2 virus infection.

## Patentansprüche

1. Eine SARS-CoV-2-virusprotektive Formulierung in Form einer Lutschtablette oder einer Kautablette, **dadurch gekennzeichnet, dass** sie Hilfsstoffe und eine Schutzmischung aus vier Extrakten umfasst, wobei die Gewichtsanteile der Schutzmischung aus vier Extrakten wie folgt sind:
- 10-55 Gew.-% Grüntee-Extrakt (6.1), der mindestens 10 % EGCG als Wirkstoff enthält,
- 10-50 Gew.-% Heidelbeer-Extrakt (11), der mindestens 0,75 % Gesamt-Anthocyanin als Wirkstoff enthält,
- 10-55 Gew.-% Granatapfelschalen-Extrakt (14), der mindestens 0,2 % EA als Wirkstoff enthält,
- 10-50 Gew.-% Schwarzpappel-Propolis-Extrakt (17), der mindestens 0,65 % CAPE als Wirkstoff enthält.

2. Eine Lutschtablette oder eine Kautablette nach Anspruch 1, **dadurch gekennzeichnet, dass** die Gewichtsverhältnisse von Grüntee-Extrakt, Heidelbeer-Extrakt, Granatapfelschalen-Extrakt und Schwarzpappel-Propolis-Extrakt darin jeweils 3(±1,5):4(±2):3(±1,5):4(:1:2) betragen.

3. Eine Lutschtablette oder eine Kautablette nach Anspruch 1 und Anspruch 2, **dadurch gekennzeichnet, dass** die Dosierung pro Einheit 20-100 mg für den Grüntee-Extrakt, 35-100 mg für den Heidelbeer-Extrakt, 20-50 mg für den Granatapfelschalen-Extrakt und 35-75 mg für den Schwarzpappel-Propolis-Extrakt beträgt.

4. Eine Lutschtablette oder eine Kautablette nach Anspruch 1, **dadurch gekennzeichnet, dass** sie mindestens einen oder mehrere der folgenden Hilfsstoffe umfasst: Sorbit (E420), Ascorbinsäure (E300), Zitronensäure (E330), Weinsäure (E334), Magnesiumsalze von Fettsäuren (E470b), mikrokristalline Cellulose (E460i), Maltodextrin, Gummi arabicum (E414), Lecithin, Rindergelatine (E441), Polyethylenglykol (E1521), Glycerin, Carboxymethylcellulose (E466), Hydroxypropylcellulose (E463), Laktose, Saccharose, Sucralose (E955), Saccharose, Xylose, Glycerol (E422), Natriumchlorid, natürliche Aromastoffe (wie Minze, Zitronenaroma) und natürliche Farbstoffe (wie Beta-Carotin und Lycopin).

5. Ein Herstellungsverfahren für eine Lutschtablette oder eine Kautablette nach Anspruch 1, **dadurch gekennzeichnet, dass** es die folgenden Schritte umfasst:
- Grüntee-Extraktion (A)
- Heidelbeer-Extraktion (D)
- Granatapfelschalen-Extraktion (F)
- Schwarzpappel-Propolis-Extraktion (G)
- Mischen des in Schritt (A) hergestellten Grüntee-Extrakts (6.1), des in Schritt (D) hergestellten Heidelbeer-Extrakts (11), des in Schritt (F) hergestellten Granatapfelschalen-Extrakts (14) und des in Schritt (G) hergestellten Schwarzpappel-Propolis-Extrakts (17) in den in Anspruch 1 angegebenen Verhältnissen (18)
- Zugabe von Hilfsstoffen zu der resultierenden Mischung (18),
- Überführen der Mischung in die Form einer Lutschtablette oder einer Kautablette.

6. Eine SARS-CoV-2-virusprotektive Formulierung in Form einer Lutschtablette oder einer Kautablette nach einem der Ansprüche 1 bis 4 zur Verwendung zur Prävention und/oder zur Behandlung einer SARS-CoV-2-Virusinfektion.

## Revendications

1. La Formulation protectrice contre le virus SARS-CoV-2 sous forme de pastille ou de comprimé à croquer, **caractérisée en ce qu'**elle comprend des excipients et un mélange protecteur de quatre extraits, les quantités en poids du mélange protecteur des quatre extraits sont les suivantes:
- 10 à 55 % en poids d'extrait du thé vert (6.1) contenant au moins 10 % d'EGCG comme ingrédient actif,
- 10 à 50 % en poids d'extrait de myrtille (11) contenant au moins 0,75 % d'anthocyanes totales comme ingrédient actif,
- 10 à 55 % en poids d'extrait d'écorce de grenade (14) contenant au moins 0,2 % d'EA comme ingrédient actif,
- 10 à 50 % en poids d'extrait de propolis de peuplier noir (17) contenant au moins 0,65 % de CAPE comme ingrédient actif.

2. Une pastille ou un comprimé à croquer selon la demande 1, **caractérisé en ce que** les rapports pondéraux d'extrait du thé vert, d'extrait de myrtille, d'extrait d'écorce de grenade et d'extrait de propolis de peuplier noir qui le composent sont respectivement de 3(±1,5):4(±2):3(±1,5):4(±2).

3. Une pastille ou un comprimé à croquer, selon la demande 1 et la demande 2, **caractérisé en ce que** le dosage par unité est de 20 à 100 mg pour l'extrait du thé vert, de 35 à 100 mg pour l'extrait de myrtille, de 20 à 50 mg pour l'extrait d'écorce de grenade et de 35 à 75 mg pour l'extrait de propolis de peuplier noir.

4. Une pastille ou un comprimé à croquer selon la demande 1, **caractérisé en ce qu'**il comprend au moins un ou plusieurs des excipients suivants : sorbitol (E420), acide ascorbique (E300), acide citrique (E330), acide tartrique (E334), sels de magnésium d'acides gras (E470b), cellulose microcristalline (E460i), maltodextrine, gomme arabique (E414), lécithine, gélatine bovine (E441), polyéthylène glycol (E1521), glycérine, carboxyméthylcellulose (E466), hydroxypropylcellulose (E463), lactose, saccharose, sucrolose (E955), saccharose, xylose, glycérol (E422), chlorure de sodium, arômes naturels (tels que menthe, arôme citron) et colorants naturels (tels que bêta-carotène et lycopène).

5. Le Procédé de fabrication d'une pastille ou d'un comprimé à croquer selon la demande 1, caractérisé en comprenant les étapes suivantes :
- Extraction du thé vert (A)
- Extraction de myrtille (D)
- Extraction de la peau de grenade (F)
- Extraction de propolis de peuplier noir (G)
- Le mélange de l'extrait du thé vert (6.1) produit à l'étape (A), l'extrait de myrtille (11) produit à l'étape (D), l'extrait d'écorce de grenade (14) produit à l'étape (F) et l'extrait de propolis de peuplier noir (17) produit à l'étape (G) sont obtenus dans les proportions indiquées dans la demande 1 (18)
- L'ajout des excipients au mélange obtenu (18)
- Mettre le mélange sous forme de pastille ou de comprimé à croquer.

6. Une formulation protectrice contre le virus SARS-CoV-2 ; l'une quelconque de celles énumérées aux demandes 1 à 4, sous forme de pastille ou de comprimé à croquer, à utiliser dans la prévention et/ou le traitement de l'infection virale SARS-CoV-2.
